# EUROPEAN PATENT APPLICATION

(11) **EP 4 755 431 A2**
(43) Date of publication of application: **10.06.2026**
(21) Application number: 26159000.4
(22) Date of filing: 28.07.2020
(51) Int. Cl.: A61M 16/06

(54) **SYSTEMS AND METHODS FOR TAILORING THERAPY PROVIDED BY RESPIRATORY SYSTEMS BASED ON SLEEP STAGE**

(30) Priority: 31.07.2019 US 201962881199 P
(62) Divisional of application: 20758002.8
(71) Applicant: ResMed Pty Ltd, Bella Vista, New South Wales 2153 (AU)
(72) Inventor: WEALE, Andrew, John, Bella Vista, 2153 (AU)
(74) Representative: Mathys & Squire

(57) **Abstract**

A system includes a respiratory device, a mask, a sensor, and a control system. The respiratory device is configured to supply pressurized air. The mask is coupled to the respiratory device and configured to engage a user during a sleep session to aid in directing the supplied pressurized air to the user. The sensor is configured to generate physiological data associated with the user. The control system is configured to analyze the physiological data to determine a first sleep stage of the user, and based on the determined first sleep stage of the user, (i) set a range of pressures for the respiratory device to supply the pressurized air, and (ii) set a rate of change of the pressurized air for the respiratory device to use when changing the supplied pressurized air from a first pressure within the range of pressures to a second pressure within the range of pressures.

## Description

### CROSS-REFERENCE TO RELATED APPLICATIONS

This application claims the benefit of and priority to U.S. Provisional Application No. 62/881,199, filed July 31, 2019, which is hereby incorporated by reference herein in its entirety.

### TECHNICAL FIELD

The present disclosure relates to treatment of respiratory-related disorders and more specifically to systems and methods for detecting sleep stages of a user and tailoring therapy on respiratory devices and their components based on the determined sleep stage.

### BACKGROUND

Various systems exist for aiding users experiencing sleep apnea and related respiratory disorders. A range of respiratory disorders exist that can impact users. Certain disorders are characterized by particular events (e.g., apneas, hypopneas, hyperpneas, or any combination thereof). Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), and Chest wall disorders. A person with respiratory disorder can have trouble sleeping, but systems designed to mitigate physical symptoms of the respiratory disorder can end up disturbing the person's sleep, having the person wake due to the applied therapy.

Thus, a need exists for alternative systems and methods for addressing sleep disturbances related to treatments for respiratory disorders. The present disclosure is directed to solving these problems and addressing other needs.

### SUMMARY

According to some implementations of the present disclosure, a method includes receiving, from a sensor, physiological data associated with a user. A first sleep stage of the user is determined based on the received physiological data. Based on the determined first sleep stage of the user, a range of pressures for a respiratory device to supply pressurized air is set. The respiratory device is coupled to a mask configured to engage the user during a sleep session to aid in directing the supplied pressurized air to an airway of the user. Based on the determined first sleep stage of the user, a rate of change of the pressurized air is set. The respiratory device uses the rate of change when changing the supplied pressurized air from a first pressure within the range of pressures to a second pressure within the range of pressures.

According to some implementations of the present disclosure, a method includes receiving, from a sensor, physiological data associated with a user. The physiological data is analyzed to determine (i) a current sleep stage of the user and (ii) a current breathing pattern of the user. A likelihood that the user will experience an apnea event within a predetermined amount of time is determined based at least in part on the determined current sleep stage and the determined current breathing pattern. Based at least in part on the determined likelihood, a respiratory device supplying pressurized air is caused to change a current pressure of the supplied pressurized air to a second pressure. The respiratory device changes the current pressure according to a rate of change based at least in part on the determined current sleep stage of the user. The respiratory device is coupled to a mask configured to engage the user to aid in directing the supplied pressurized air to an airway of the user.

According to some implementations of the present disclosure, a system for setting a pressure range and a rate of change of pressure based on a determined sleep stage is provided. The system includes a respiratory device, a mask, a sensor, a memory, and a control system. The respiratory device is configured to supply pressurized air. The mask is coupled to the respiratory device via a tube and is configured to engage a user during a sleep session to aid in directing the supplied pressurized air to an airway of the user. The sensor is configured to generate physiological data associated with the user. The memory is configured to store machine-readable instructions. The control system includes one or more processors configured to execute the machine-readable instructions to: analyze the physiological data to determine a first sleep stage of the user; based on the determined first sleep stage of the user, set a range of pressures for the respiratory device to supply the pressurized air; and based on the determined first sleep stage of the user, set a rate of change of the pressurized air for the respiratory device to use when changing the supplied pressurized air from a first pressure within the range of pressures to a second pressure within the range of pressures.

According to some implementations of the present disclosure, a system for adjusting pressure based on a determined likelihood of a future apnea event is provided. The system includes a respiratory device, a mask, a sensor, a memory, and a control system. The respiratory device is configured to supply pressurized air. The mask is coupled to the respiratory device via a tube and is configured to engage a user during a sleep session to aid in directing the supplied pressurized air to an airway of the user. The sensor is configured to generate physiological data associated with the user. The memory is configured to store machine-readable instructions. The control system includes one or more processors configured to execute the machine-readable instructions to: analyze the physiological data to determine (i) a current sleep stage of the user and (ii) a current breathing pattern of the user; determine a likelihood that the user will experience an apnea event within a predetermined amount of time based at least in part on the determined current sleep stage and the determined current breathing pattern; and based at least in part on the determined likelihood, cause the respiratory device to change, according to a rate of change based at least in part on the determined current sleep stage of the user, a current pressure of the supplied pressurized air to a second pressure.

According to some implementations of the present disclosure, a system for training a machine learning algorithm to determine a likelihood of a future apnea event is provided. The system includes a respiratory device, a mask, a sensor, a memory, and a control system. The respiratory device is configured to supply pressurized air. The mask is coupled to the respiratory device via a tube and is configured to engage a user during a sleep session to aid in directing the supplied pressurized air to an airway of the user. The sensor is configured to generate physiological data associated with the user. The memory is configured to store machine-readable instructions. The control system includes one or more processors configured to execute the machine-readable instructions to: accumulate the physiological data, the physiological data including historical physiological data and current physiological data; and train a machine learning algorithm with the historical physiological data such that the machine learning algorithm is configured to receive as an input the current physiological data and determine as an output a likelihood that the user will experience an apnea event within a predetermined amount of time.

According to some implementations of the present disclosure, a method of supplying pressurized air to a user of a respiratory device is provided. A respiratory device supplies pressurized air at a first pressure to the user via a mask coupled to the respiratory device, where the first pressure is within a first range of pressures. A sensor generates first physiological data associated with a first portion of a sleep session of the user. A first sleep stage of the user is determined based at least in part on the first physiological data. Based on the determined first sleep stage of the user, the first pressure is changed according to a first rate of change to a second pressure such that the second pressure of the supplied pressurized air is within the first range of pressures. The sensor generates second physiological data associated with a second portion of the sleep session of the user. A second sleep stage of the user is determined based at least in part on the second physiological data, where the second sleep stage is different than the first sleep stage. Based on the determined second sleep stage of the user, the second pressure is changed according to a second rate of change to a third pressure such that the third pressure of the supplied pressurized air is within a second range of pressures that is different than the first range of pressures, where the first rate of change and the second rate of change have different magnitudes.

The foregoing and additional aspects and implementations of the present disclosure will be apparent to those of ordinary skill in the art in view of the detailed description of various embodiments and/or implementations, which is made with reference to the drawings, a brief description of which is provided next.

### BRIEF DESCRIPTION OF THE DRAWINGS

The foregoing and other advantages of the present disclosure will become apparent upon reading the following detailed description and upon reference to the drawings.
FIG. 1 illustrates an environment for using a respiratory device, according to some implementations of the present disclosure;
FIG. 2 is a perspective view of a mask or mask assembly for use with a respiratory device, according to some implementations of the present disclosure;
FIG. 3 is a block diagram of a system for adjusting pressure of a respiratory system based on sleep stage, according to some implementations of the present disclosure;
FIG. 4 is a flow diagram for configuring settings of a respiratory system based on sleep stage, according to some implementations of the present disclosure;
FIG. 5 is a flow diagram for monitoring and adjusting configuration of a respiratory system based on sleep stage, according to some implementations of the present disclosure;
FIG. 6A illustrates an example where maximum pressure provided by a respiratory system differs between sleep stages, according to some implementations of the present disclosure;
FIG. 6B illustrates an example where rate of increasing pressure in response to an apnea event differs between sleep stages, according to some implementations of the present disclosure;
FIG. 6C illustrates an example where minimum pressure provided by a respiratory system differs between sleep stages, according to some implementations of the present disclosure;
FIG. 6D illustrates an example where rate of decreasing pressure differs between sleep stages, according to some implementations of the present disclosure;
FIG. 7 is a flow diagram for monitoring and adjusting configuration of a respiratory system based on a likelihood of its user having an apnea event, according to some implementations of the present disclosure;
FIG. 8 illustrates an example placement of components for adjusting pressure of a respiratory system based on sleep stage, according to some implementations of the present disclosure; and
FIG. 9 illustrates a perspective view of a mask or mask assembly that can be used with a respiratory device to adjust pressure of the respiratory system based on sleep stage, according to some implementations of the present disclosure.

While the present disclosure is susceptible to various modifications and alternative forms, specific implementations have been shown by way of example in the drawings and will be described in detail herein. It should be understood, however, that the present disclosure is not intended to be limited to the particular forms disclosed. Rather, the present disclosure is to cover all modifications, equivalents, and alternatives falling within the spirit and scope of the present disclosure as defined by the appended claims.

### DETAILED DESCRIPTION

Many individuals suffer from sleep-related and/or respiratory disorders. Examples of sleep-related and/or respiratory disorders include Periodic Limb Movement Disorder (PLMD), Restless Leg Syndrome (RLS), Sleep-Disordered Breathing (SDB), Obstructive Sleep Apnea (OSA), apneas, Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as central apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

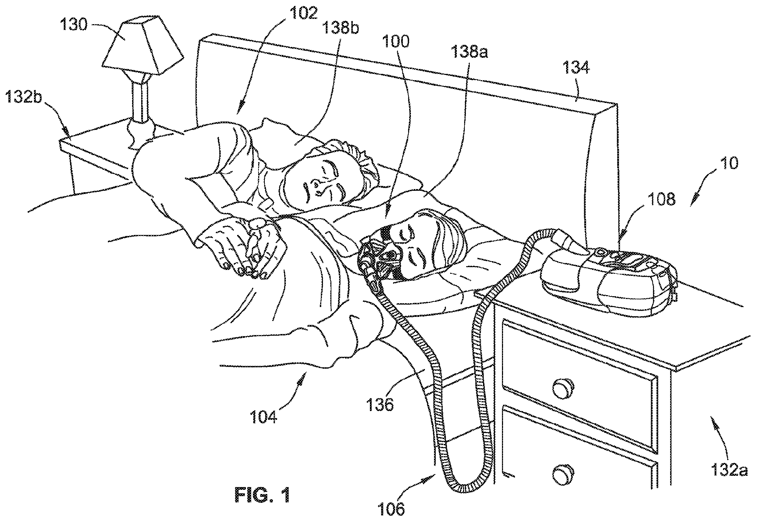

FIG. 1 illustrates an environment (e.g., a bedroom) for using a respiratory device, system, and/or any of its components (e.g., mask, motor, water tank, pump, display, etc.), according to some implementations of the present disclosure. The environment is a bedroom setting that includes a user 104 (e.g., a user of a respiratory system) and a bed partner 102 lying on a bed with a mattress 136, pillows 138a and 138b, and a headboard 134. Next to the bed are two nightstands 132a and 132b, and a lamp 130 on the nightstand 132b. The user 104 is wearing a user interface (e.g., a mask 100) connected via an air circuit or a conduit (e.g., a tube or tubing 106) to a respirator or respiratory device 108. The mask 100, the air circuit or tubing 106, and the respiratory device 108 are collectively referred to as a respiratory system 10.

The user 104 may suffer from obstructive sleep apnea and relies on the mask 100 to deliver pressurized air from the respiratory device 108 via the tubing 106. The respiratory device 108 can be a continuous positive airway pressure (CPAP) machine used to increase air pressure in the throat of the user 104 to prevent the airway from closing and/or narrowing during sleep. For someone with sleep apnea, her airway can narrow or collapse during sleep, reducing oxygen intake, and forcing her to wake up and/or otherwise disrupt her sleep. The CPAP machine aids in preventing the airway from narrowing or collapsing, thus minimizing the occurrences where she wakes up or is otherwise disturbed (e.g., due to reduction in oxygen intake, choking, coughing, snoring, etc. or any combination thereof).

Although CPAP is used as an example, the respiratory device 108 can be used as, for example, as a ventilator or a positive airway pressure (PAP) system such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

The respiratory device 108 strives to maintain a medically prescribed air pressure during sleep, but in some cases, a physician roughly estimates and prescribes a large pressure range for the medically prescribed air pressure. The respiratory system 10 of the present disclosure can implement therapy with a large prescribed pressure range while accounting for the sensitivity of the user 104 to the air pressure during different stages of sleep. For example, some pressure levels within the large pressure range are more appropriate when the user 104 is in a rapid eye movement (REM) sleep stage than in other sleep stages. As such, the respiration system 10 can implement and/or supply the large pressure range prescribed by the physician in a customized manner to the needs of the user 104. The customized pressure range and/or ability to adjust pressure throughout a sleep session allows the user 104 to have a higher overall quality of sleep, minimizing disturbances due to the supplied pressure of the respiratory device 108 during the sleep session.

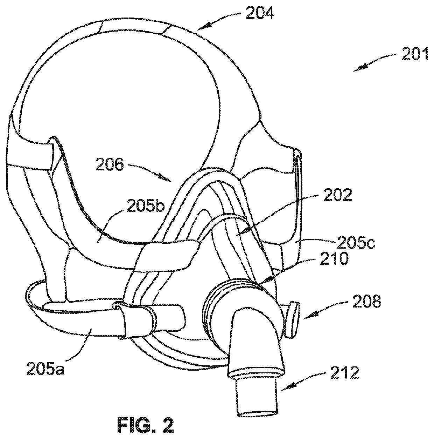

Referring to FIG. 2, a mask 201 for use with a respiratory device (e.g., the respiratory device 108) is shown according to some implementations of the present disclosure. The mask 201, is the same as, or similar to, the mask 100 of FIG. 1. The mask 201 includes a plenum chamber 202, a positioning or stabilizing structure 204 with strap segments 205a, 205b, and 205c, a cushion 206, an electronics interface 208, a connecting section 210, and a connection port or coupling mechanism 212. The positioning or stabilizing structure 204 allows a user, e.g., the user 104, to affix the plenum chamber 202 snugly to her face. The cushion 206 is provided between the face of the user 104 and the plenum chamber 202 to improve comfort to the wearer and also to act as a seal or a conforming membrane for preventing pressurized air from leaking out of the plenum chamber 202 at the interface between the face of the user 104 and the plenum chamber 202. The cushion 206 can be made from silicone, plastic, foam, or any combination thereof.

The connecting section 210 allows attachment of the connection port 212 to the plenum chamber 202. The connection port 212 mates with a tubing, e.g., the tubing 106, for receiving pressurized air from the respiratory device 108. The connecting section 210 can provide a swivel such that the connection port 212 can be rotated to a desired position. The connection section 210 can also provide controlled vents to allow carbon dioxide and other gases exhaled by the user 104 to escape the plenum chamber 202. The controlled vents can allow continuous vent flow from an interior of the plenum chamber 202 to ambient whilst the pressure within the plenum chamber 202 is positive with respect to the ambient. The controlled vents at the connection section 210 are structured such that the vent flowrate has a magnitude sufficient to reduce rebreathing of exhaled carbon dioxide by the user 104, while maintaining the therapeutic pressure in the plenum chamber 202.

In some implementations, the mask 201 includes one or more supplementary ports that allow access to volume within the plenum chamber 202. The supplementary port(s) can allow a clinician or physician to probe or access property of gases within the plenum chamber 202. For example, the supplemental port can aid in determining pressure within the plenum chamber 202. The electronics interface 208 provides connection to electronic sensors and other devices that can be embedded in the mask 201. The mask 201 can include a breathalyzer sensor for determining blood alcohol content of the user 104.

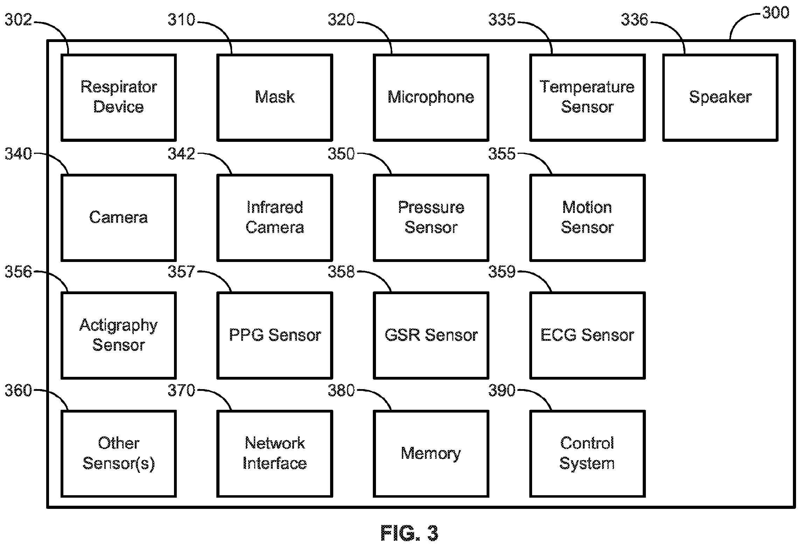

Referring to FIG. 3, a block diagram of a system 300 for adjusting pressure provided by a respiratory device 302 is shown according to some implementations of the present disclosure. The system 300 measures physiological data, and adjusts the pressure of the air provided by the respiratory device 302. The physiological data is, for example, indicative of a status of the user 104 and/or one or more aspects/attributes of the user 104. To simplify discussion, the singular form will be used for all components identified in FIG. 3 when appropriate, but the use of the singular does not limit the discussion to only one of each such component.

The respiratory device 302 is the same as, or similar to, the respiratory device 108 and can be, for example, a CPAP machine. The mask 310 is the same as, or similar to the mask 201 and/or the mask 100. The mask 310 can be worn by or donned upon the user 104 (FIG. 1). In some implementations, the mask 310 includes the connection section 210 (FIG. 2) which includes vents designed to allow exhaled gases to escape the plenum chamber 202. The respiratory device 302 can be configured to generate a flow of air for delivery to the airways of the user 104. The respiratory device 302 can deliver air flow in a range of -20 L/min to 150 L/min while maintaining a positive pressure of at least 6 cm H₂0, or at least 10 cm H₂0, or at least 20 cm H₂0. The respiratory device 302 can include an external housing with one or more panels, a handle, or any combination thereof.

The respiratory device 302 can also include an inlet air filter, an inlet muffler, a pressure generator for supplying air at positive pressure, an outlet muffler, and one or more transducers, e.g., pressure sensors and flowrate sensors. The respiratory device 302 can have an electrical power supply, one or more input devices (e.g., buttons, dials, switches, touchscreens, and so on), and a central controller. In some implementations, the respiratory device 302 includes a humidifier and an anti-spill back valve that reduces risk that water will flow from the respiratory device 302 to the user 104 (e.g., via the tubing 106 shown in FIG. 1).

The respiratory device 302 can further include a wired or wireless data communication interface for communicating with electronic components or sensors on the mask 201. In some implementations, the tubing 106 not only carries pressurized air but also includes an electric wire for connecting the data communication interface on the respiratory device 302 to sensors and/or one or more electronic components (e.g., sensors, microphones, cameras, memory, control systems, etc., or any combination thereof) built into and/or coupled to the mask 310.

The system 300 can further include a microphone 320 for sensing sound in its vicinity. The microphone 320 can be wired or wireless and can be positioned at any place in a room containing the system 300 (e.g., in the bedroom shown in FIG. 1). The microphone 320 can also be positioned somewhere on and/or in the respiratory device 302, the mask 310, or both. In some implementations, the microphone 302 is referred to as a passive monitoring system in that the microphone passively listens for sounds.

The system 300 can further include, along with the microphone 320, a speaker 336 such that the microphone 320 and the speaker 336 collectively provide a sonar sensor for the system 300. The speaker 336 can produce sound at intervals, and the microphone 320 can be used to listen for echoes of the sound when the speaker 336 is not producing sound. That way, the microphone 320 and the speaker 336 implement a sonar system that can be used to detect movement of the user 104. In some implementations, the speaker 336 produces sound at inaudible frequencies (e.g., below twenty hertz or above twenty kilohertz) so as to not disturb the user 104 and/or a bed partner of the user 104. In some implementations, the speaker 336 produces sound at the audible frequencies between twenty hertz and twenty kilohertz.

The system 300 can further include a temperature sensor 335, a camera 340, an infrared camera 342, a pressure sensor 350, a motion sensor 355, an actigraphy sensor 356, a photoplethysmogram (PPG) sensor, a galvanic skin response (GSR) sensor, electrocardiogram (ECG) sensor, and other sensors 360 (e.g., electroencephalography (EEG) sensor, electromyography (EMG) sensor, blood flow sensor, respiration sensor, pulse sensor, sphygmomanometer sensor, oximetry sensor, a moisture sensor, etc.). The temperature sensor 335 can be used to determine temperature of the user 104 at various locations on the body of the user 104. The camera 340 and infrared camera 342 can be positioned to capture movement and change in heat signatures of the user 104. The pressure sensor 350 can be located anywhere along the air circuit from the respiratory device 302 to the mask 310. The pressure sensor 350 can be multiple pressure sensors positioned along the air circuit to measure pressure at different points within the air circuit (e.g., at the plenum chamber of the mask, along the tubing connecting the mask to the respiratory device, at either end of the tubing, at the respiratory device 302, and so on).

The motion sensor 355 can detect movement of the user 104. In some implementations, the motion sensor 355 cooperates with the infrared camera 342 to determine changes and/or shifts in body temperature with respect to ambient temperature to determine whether a person is moving. In some implementations, the motion sensor 355 utilizes electromagnetic sensing in the infrared wavelength for detecting motion and determines that body temperature slightly falls while an individual is sleeping so when body temperature rises above a certain level based on infrared sensing, then the motion sensor 355 determines that the individual is waking up and/or moving. Other examples of the motion sensor 355 include passive infrared sensors, sensors that emit ultrasonic signals (as described above) and determine whether detected reception of reflected ultrasonic signals indicate a changing pattern, gyroscopes and accelerometers embedded in pajamas or beddings, passive microphones, sonar sensors, radar sensors, ultra wide band sensors, and so on.

The motion sensor 355 can include radar sensors and/or ultra wide band sensors. Radar sensors involve using one or more transmitters that produce radio waves and one or more receivers that detect reflected radio waves to determine position, orientation, and/or movement of the user 104 and/or of any item or thing or person or pet, etc. in a room and/or house/building. Radio waves produced can be in any radio frequency (RF) band, for example, high frequency band, very high frequency band, long wave, short wave, and so on. In some implementations, the frequency band chosen can also be in crowded bands such as medium frequency band, low frequency band, and so on. Ultra wide band sensors are similar to radar sensors except ultra wide band sensors transmit radio waves over a larger bandwidth compared to typical radar sensors. The motion sensor 355 can include ultra wide band transmitters and receivers to realize an ultra-wide band sensor.

The actigraphy sensor 356 generates physiological data indicative of movement of the user 104 by monitoring body movements of the user 104. The actigraphy sensor 356 can be worn on a body part of the user 104, such as, for example, worn on the wrist, ankle, neck, and so on. The actigraphy sensor 356 can include a band worn on the chest for detecting chest movement. The actigraphy sensor 356 can include an accelerometer for measuring acceleration of the body part of the user 104. The actigraphy sensor 356 can also include a timer and processor for recording and accumulating accelerometer values at specific times and/or at specific intervals. The actigraphy sensor 356 can also include filters to remove tremors and/or vibrations.

In some implementations, the system 300 includes the PPG sensor 357. The PPG sensor 357 can be positioned next to the neck, temple, forehead, head, wrist, arm, or some other body part of the user 104. The PPG sensor 357 can generate physiological data that is indicative of blood flow of the user 104, blood oxygen levels of the user 104, heart rate of the user 104, an apnea event the user 104 is currently experiencing, an apnea event the user 104 is likely to experience in the future, or any combination thereof.

In some implementations, the system 300 includes the GSR sensor 358. The GSR sensor 358 can be positioned anywhere on the body of the user 104 or specifically in areas of the body of the user 104 with greater sweat gland activity, such as, for example, on the palms, on the fingers, on the feet, or on the forehead of the user 104. The GSR sensor 358 can generate physiological data that is indicative of electrical properties of the skin which can, in some implementations, be used to determine an emotional arousal of the user 104.

In some implementations, the system 300 includes the ECG sensor 359. The ECG sensor 359 can have electrodes that are incorporated in a band (e.g., a band worn around the chest of the user 104) to generate physiological data indicative of heart activity. The ECG sensors 359 can be used to generate heart rate data of the user 104, heart rate variability of the user 104, and so on. In some implementations, the ECG sensor 359 is an ECG sensor with electrodes that can be capacitively coupled to the chest of the user 104.

In some implementations, the mask 310 includes an EEG sensor for generating physiological data indicative of brain electrical activity of the user 104. In some implementations, the EEG sensor is separate from the mask 310 and can be incorporated in a headphone worn by the user 104. In some implementations, the EEG sensor is a non-contact sensor that can be coupled to the scalp of the user 104, e.g., via capacitive coupling.

The memory 380 can include one or more physically separate memory devices, such that one or more memory devices can be coupled to and/or built into the respiratory device 302, the control system 390, and/or one or more external devices (e.g., mobile phones, computers, servers, cloud based devices, etc.) wirelessly coupled and/or wired to the system 300. The memory 380 acts as a non-transitory computer readable storage medium on which is stored machine-readable instructions that can be executed by the control system 390 and/or one or more other systems. The memory 380 is also able to store (temporarily and/or permanently) the physiological data generated by sensors of the system 300. In some implementations, the memory 380 includes non-volatile memory, battery powered static RAM, volatile RAM, EEPROM memory, NAND flash memory, or any combination thereof. In some implementations, the memory 380 is a removable form of memory (e.g., a memory card).

Like the memory 380, the network interface 370 can be coupled to the respiratory device 302, the mask 310, the control system 390, and/or one or more external devices. The network interface 370 is coupled to the memory 380 such that the control system 390 is configured to communicate with one or more external devices or other components in the system 300.

Also like the memory 380, the control system 390 can be coupled to the respiratory device 302, the mask 310, and/or one or more external devices. The control system 390 is coupled to the memory 380 such that the control system 390 is configured to execute the machine-readable instructions stored in the memory 380. The control system 390 can include one or more processors and/or one or more controllers. In some implementations, the one or more processors includes one or more x86 INTEL processors, one or more processors based on ARM^{®} Cortex^{®}-M processor from ARM Holdings such as an STM32 series microcontroller from ST MICROELECTRONIC, or any combination thereof. In some implementations, the one or more processors include a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS.

In some implementations, the control system 390 is a dedicated electronic circuit. In some implementations, the control system 390 is an application-specific integrated circuit. In some implementations, the control system 390 includes discrete electronic components.

The control system 390 is able to receive input(s) (e.g., signals, generated data, instructions, etc.) from any of the other elements of the system 300 (e.g., the sensors, etc.). The control system 390 is able to provide output signal(s) to cause one or more actions to occur in the system 300 (e.g., to cause the respiratory device 302 to provide pressurized air at a specific pressure, etc.).

While the control system 390 and the memory 380 are described and shown in FIG. 3 as being a separate and distinct component of the system 300, in some implementations, the control system 390 and/or the memory 380 are integrated in the respiratory device 302. Alternatively, in some implementations, the control system 390 or a portion thereof (e.g., at least one processor of the control system 390) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While the system 300 is shown as including all of the components described above, more or fewer components can be included in a system according to implementations of the present disclosure. For example, a first alternative system includes the control system 390, the memory 380, and at least one of the sensors provided in FIG. 3. As another example, a second alternative system includes the control system 390, the memory 380, the respiratory system 302, and at least one of the sensors provided in FIG. 3. Thus, various systems can be formed using any portion or portions of the components shown and described herein and/or in combination with one or more other components.

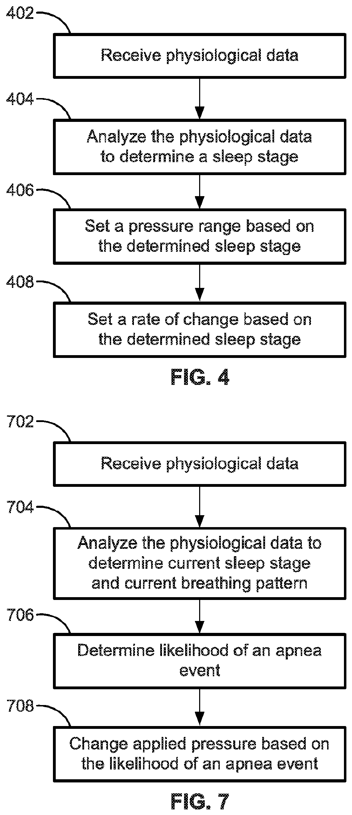

Referring to FIG. 4, a flow diagram illustrating a method for configuring settings of a respiratory system (e.g., the respiratory device 302) based on sleep stage is shown according to some implementations of the present disclosure. At step 402, the control system 390 (and/or the respiratory device 302 in some implementations) receives physiological data. The physiological data can come from the respiratory device 302 generating pressure data with the pressure sensor 350. The pressure data generated can be used to determine other physiological data including respiration rate, apneas, etc. The physiological data can come from the microphone 320 generating sound data while the user 104 is awake, is trying to fall asleep, or is asleep. The physiological data can come from the microphone 320 and a speaker 336 combination working as a sonar sensor to generate movement data that indicates movement of the user 104. The physiological data can come from transmitters and receivers acting as a radar sensor and/or an ultra-wide band sensor to generate movement data indicating movement of the user 104.

In some implementations, the physiological data comes from one or more of the microphones 320, one or more of the pressure sensors 350, one or more of the infrared cameras 342, one or more of the cameras 340, one or more actigraphy sensors 356, one or more PPG sensors 357, one or more GSR sensors 358, one or more ECG sensors 359, one or more ECG sensors, one or more flow rate sensors, one or more of the other sensors 360, or any combination thereof. Examples of the physiological data can include respiration rate, breath analysis, airway resistance, blood flow to brain, blood pressure, skin temperature, core temperature, brain activity, heart rate, muscle tone, sexual arousal, sympathetic nerve activity, blood alcohol level, activity (body movement, chest movement, limb movement, body position, coughing in bed), blood oxygen saturation (SpO2), expired carbon dioxide (CO₂), or any combination thereof.

At step 404, the control system 390 analyzes the physiological data to determine a sleep stage associated with the user 104. In some implementations, the control system 390 determines whether the user 104 is in a REM sleep stage or a non-REM (NREM) sleep stage. In some implementations, the control system 390 determines whether the user 104 is in one of three sleep stages including wakefulness, REM, and NREM. In some implementations, the control system 390 determines whether the user 104 is in one of four sleep stages including wakefulness, light sleep, deep sleep, and REM. In some implementations, the control system 390 determines whether the user 104 is in one of the following sleep stages: wakefulness, relaxed wakefulness, light sleep, deep sleep, or REM. In some implementations, the control system 390 determines whether the user 104 is in one of any number of sleep stages.

In some implementations, the control system 390 determines that wakefulness sleep stage is a baseline for evaluating the other sleep stages. During this sleep stage, the user 104 is awake, and the respiratory device 302 and/or the different sensors (e.g., the actigraphy sensor 356, the PPG sensor 357, and so on) can be used to determine physiological data for comparing with the other sleep stages.

In some implementations, the control system 390 determines that the user 104 is in a REM sleep stage. In some such implementations, the control system 390 bases its determination at least in part on a determination that the brain of the user 104 is active but the body of the user 104 is paralyzed. The physiology of REM sleep can include rapid eye movements; loss of muscle tone; low amplitude mixed frequency EEG; limited thermoregulation, shivering, or sweating; elevated heart rate; elevated respiration rate; and any combination thereof. The control system 390 can use respiration rate generated by the respiratory device 302 and/or one or more other devices, movement data generated from the camera 340, the infrared camera 342, the motion sensor 355, the actigraphy sensor 356, a sonar sensor, a radar sensor, or any combination thereof, to characterize whether the user 104 is in a REM sleep stage.

The control system 390 can use conductance data generated by the GSR sensor 358 to determine whether the user 104 is sweating. The control system 390 can determine movement of the chest using the motion sensor 355 to estimate respiration rate. The control system 390 can use heart rate data generated by the PPG sensor 357 and/or the ECG sensor 359 to determine whether heart rate of the user 104 is elevated.

In comparison with the wakefulness sleep stage, the control system 390 can determine from the respiration rate and/or respiration flow that airway resistance varies during the REM sleep stage. The control system 390 can determine from the respiration rate and heart rate data that blood flow to the brain increases during REM sleep stage. The control system 390 can determine from heart rate data and/or blood pressure data that blood pressure varies and that heart rate also varies during REM sleep stage. The control system 390 can determine through temperature data generated by the temperature sensor 335 that body temperature varies during REM sleep stage.

The control system 390 can determine through brain wave data of an EEG sensor that brain activity increases during REM sleep stage. The control system 390 can determine through movement data generated by the actigraphy sensor 356 and/or an EMG sensor that muscle tone decreases during REM sleep stage. The control system 390 can use the respiration data to determine that respiration varies during REM sleep stage. The control system 390 can determine that sexual arousal increases during REM sleep stage. The control system 390 can determine through heart rate data, movement data, and/or conductance data that sympathetic nerve activity increases during REM sleep stage.

In some implementations, the control system 390 determines that the user 104 is in a relaxed wakefulness sleep stage. In some such implementations, the control system 390 bases its determination at least in part on a determination that core body temperature of the user 104 drops at sleep onset, the user 104 has slow eye movements, and sleep spindles do not exist in brain wave data. The temperature sensor 335 and/or the infrared camera 342 can be used to obtain temperature data. An example of the temperature sensor 335 is a digital thermometer that can interface with the control system 390 and/or the respiratory device 302. The relaxed wakefulness is a sleep stage that can be about ten minutes long, and in some instances can account for about five percent of sleep time.

An electroencephalography (EEG) sensor can obtain brain wave data from the user 104, and the control system 390 can evaluate the brain wave data for sleep spindles (also known as sigma bands or sigma waves). The camera 340 can generate video data that the control system 390 can apply computer vision algorithms in order to determine eye movements of the user 104. In some implementations, hypnic jerks are present during the relaxed wakefulness sleep stage, so the actigraphy sensor 356 and/or an EMG sensor can be used to measure movements of the user 104 during this sleep stage.

In some implementations, the control system 390 determines that the user 104 is in a light sleep stage. In some such implementations, the control system 390 bases its determination at least in part on a determination that muscles of the upper airway of the user 104 are relaxing and airways of the user 104 are narrowing. The airway narrowing can change the sound of breathing/respiration which can be captured with the microphone 320 for analysis by the control system 390. The control system 390 can determine that the user 104 is in a light sleep stage when K complexes and sleep spindles are present in brain wave data. The control system 390 can determine that the user 104 is in a light sleep stage when heart rate decreases, blood pressure decreases, eye movements cease, or a combination thereof.

The control system 390 and/or respiratory device 302 can use, for example, flow sensors, the pressure sensor 350, and so on, to determine whether the airway of the user 104 is narrowing. The airway narrowing affects respiratory flow rate of the user 104, which can be determined via different flow rates reaching the mask 310. In an example, the control system 390 can determine flow rate of the respiratory device 302, which is the flow rate of air leaving the respiratory device 302. The control system 390 can further determine a total flow rate reaching the mask 310 via the air circuit, where the total flow rate is the flow rate of air and any supplementary gas introduced via any supplementary port of the mask 310 that reaches the mask 310 via the air circuit. The control system 390 can determine a vent flow rate, which is the flow rate of air leaving a vent to allow washout of exhaled gases. The control system 390 can further determine a leak flow rate, which is the flow rate of leak from the mask 310 and/or anywhere else on the air circuit. The control system 390 can also determine a respiratory flow rate, which is the flow rate of air that is received into the respiratory system of the user 104.

In some implementations, the control system 390 can determine pressure at the mask 310, the respiratory flow rate, and the leak flow rate using pressure sensors and/or one or more flow rate sensors. The control system 390 can further estimate leak flow rate and respiratory flow rate.

In some implementations, the control system 390 determines pressure at the mask 310 using the pressure sensors 350 and/or one or more flow rate sensors. The pressure sensor 350 can provide pressure leaving the respiratory device 302 and the flow rate sensors can provide flow rate of air leaving the respiratory device 302. The control system 390 can then use the pressure leaving the respiratory device 302 to estimate a drop in pressure throughout the air circuit and/or one or more portions thereof. Absent any supplementary gases introduced to the mask 310, the flow rate of the respiratory device 302 can be used as an estimate of the total flow rate reaching the mask 310.

In some implementations, the control system 390 can model the dependence of the pressure drop through the air circuit on the total flow rate reaching the mask 310 for particular air circuits to determine pressure characteristics. The control system 390 can then use the pressure characteristics to determine an estimated pressure reaching the mask 310. For example, pressure reaching the mask 310 can be determined as respiratory device pressure minus the air circuit pressure drop.

In some implementations, the control system 390 determines vent flow rate using the pressure reaching the mask 310 and estimates the vent flow rate in the mask 310. Dependence of the vent flow rate on the pressure reaching the mask 310 can be modeled apriori, and the control system 390 uses the modeled characteristic to determine particular vent flow rates for particular determined pressures reaching the mask 310.

In some implementations, the control system 390 estimates leak flow rate via the total flow rate and the vent flow rate. The leak flow rate can be estimated by the control system 390 by calculating an average of the difference between total flow rate and vent flow rate over a period sufficiently long to include several breathing cycles. The period can be, for example, 5 second, 10 seconds, 20 seconds, 30 seconds, etc.

In some implementations, the control system 390 estimates leak flow rate via the total flow rate, the vent flow rate, and the estimated pressure reaching the mask 310. The leak flow rate can be estimated by calculating a leak conductance and determining the leak flow rate to be a function of the leak conductance and the estimated pressure reaching the mask 310. The leak conductance can be calculated as the quotient of a low-pass filtered non-vent flow rate equal to the difference between total flow rate and vent flow rate and low-pass filtered square root of the pressure reaching the mask 310, where the low pass filter time constant has a value sufficiently long to include several breathing cycles. The breathing cycles can last for about 10 seconds, 20 seconds, etc. The leak flow rate can be estimated as the product of the leak conductance and a function of the pressure reaching the mask 310.

In some implementations, the control system 390 estimates the respiratory flow rate via the total flow rate, the vent flow rate, and the leak flow rate. The respiratory flow rate can be determined by subtracting the vent flow rate and the leak flow rate from the total flow rate.

To determine whether the user 104 is in the light sleep sleep stage, the control system 390 can use the respiratory flow rate as a measure of airway narrowing and for estimating breath flattening. The control system 390 can also use EEG sensors providing brain wave patterns to determine whether K complexes and sleep spindles are present. The control system 390 can use the PPG sensor 357 and/or the ECG sensor 359 to obtain heart rate and blood pressure information. The camera 340 can be used to obtain eye movement data.

In some implementations, the control system 390 determines that the user 104 is in deep sleep (or slow-wave sleep) sleep stage. In some such implementations, the control system 390 bases its determination at least in part on a determination that brain wave data presents slow-wave brainwaves, heart rate data indicates a decreased heart rate, blood pressure data indicates a decreased blood pressure, growth hormone is released, eye movements cease, or combinations thereof. Previous techniques of determining blood pressure and heart rate still apply and are not repeated. The control system 390 can determine from EEG sensors (e.g., EEG sensors in the mask 310) that brain wave patterns show slow delta waves.

Each of the NREM sleep stages (e.g., relaxed wakefulness, light sleep, and deep sleep) can be compared with the wakefulness sleep stage baseline to distinguish between NREM, REM, and wakefulness. The control system 390 can determine that airway resistance increases during the NREM sleep stages. The control system 390 can determine that blood flow to the brain decreases during the NREM sleep stages. The control system 390 can determine that blood pressure decreases during the NREM sleep stages. The control system 390 can determine that body temperature decreases during the NREM sleep stages. The control system 390 can determine that brain activity decreases during the NREM sleep stages. The control system 390 can determine that heart rate decreases during the NREM sleep stages. The control system 390 can determine that muscle tone is comparable during the NREM sleep stages. The control system 390 can determine that respiration decreases during the NREM sleep stages. The control system 390 can determine that sexual arousal is infrequent during the NREM sleep stages. The control system 390 can determine that sympathetic nerve activity decreases during the NREM sleep stages.

At step 406, the control system 390 and/or the respiratory device 302 sets a pressure range based on the determined sleep stage. For each individual, apneas can be more common in one sleep stage compared to the other sleep stages for the specific individual. Compared to the other sleep stages, the control system 390 can set the maximum pressure of the set pressure range higher in the one sleep stage where apneas are more common for the specific individual user of the system 300. The control system 390 can learn and rank the sleep stages where apneas are more likely to occur for specific users and set the pressure range accordingly.

In some implementations, apneas are more likely in REM sleep stage in more people than in any of the NREM sleep stages, so the maximum pressure of the set pressure range is higher in REM sleep than in any of relaxed wakefulness, light sleep, or deep sleep. Also, a minimum pressure of the set pressure range is higher in REM sleep than in any of relaxed wakefulness, light sleep, or deep sleep.

In some implementations, some users are more likely to have apneas in deep sleep compared to the other sleep stages such that the maximum pressure of the set pressure range is higher in deep sleep than in REM sleep stage and any of the other NREM sleep stages. Also, a minimum pressure of the set pressure range is higher in deep sleep than in any of the other sleep stages. Examples of ranges include 6-10 cm H₂O, 7-12 cm H₂O, and so on.

At step 408, the control system 390 and/or the respiratory device 302 sets a rate of change based on the determined sleep stage. The rate of change is a rate of change that the respiratory device 302 uses when changing pressure of the supplied pressurized air to the user 104 from a first pressure within the set pressure range to a second pressure within the set pressure range. In some implementations, the rate of change is higher in REM sleep stage than in any of the NREM sleep stages. In some implementations, the rate of change is merely different in REM sleep stage than in any of the NREM sleep stages. Example rates of changes include 0.1 cm H₂O/s, 1 cm H₂O/s, 2 cm H₂O/s, and so on.

In some implementations, the control system 390 measures an amount of breath flattening of the user and when the breath flattening reaches a predetermined threshold, causes the respiratory device 302 to change supplied pressurized air from the first pressure to the second pressure according to the set rate of change. In some implementations, the control system 390 measures a magnitude of snoring with the microphone 320 and causes the respiratory device 302 to change from the first pressure to the second pressure based on the snoring event exceeding a predetermined volume threshold. In some implementations, the control system 390 detects an occurrence of an apnea of the user and at least in part in response thereto, the control system 390 causes the respiratory device to change the supplied pressurized air from the first pressure to the second pressure according to the set rate of change of the pressured air.

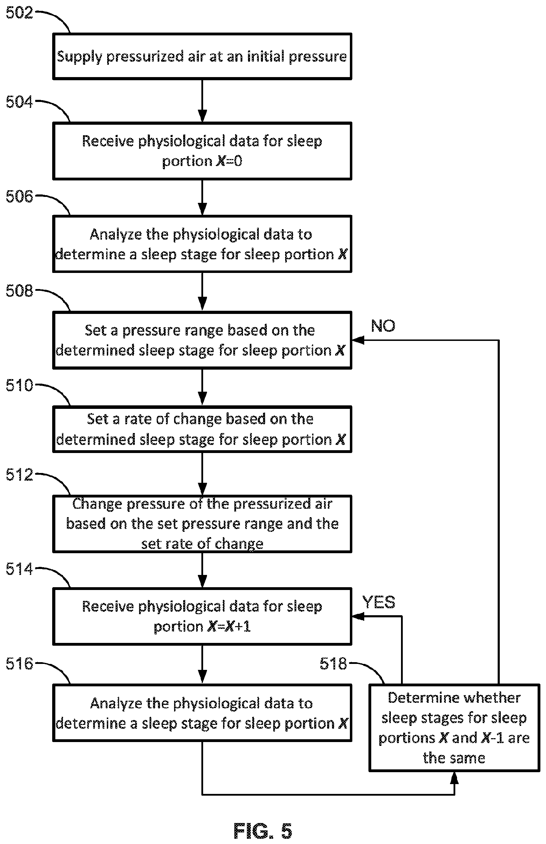

Referring to FIG. 5, a flow diagram illustrating a method for supplying pressurized air to the user 104 based on a sleep stage of the user 104 is shown according to some implementations of the present disclosure. In FIG. 5, the sleep session of the user 104 is segmented into multiple sleep portions as physiological data is continually obtained throughout the sleep session. At step 502, the control system 390 causes the respiratory device 302 to supply air at an initial pressure. At step 504, the control system 390 receives physiological data for an initial sleep portion designated as sleep portion X=0. At step 506, the control system 390 analyzes the physiological data to determine a sleep stage for the sleep portion X=0. Steps 504 and 506 are the same as, or similar to, steps 402 and 404 described above in connection with FIG. 4.

At step 508, the control system 390 sets a pressure range based on the determined sleep stage for sleep portion X. At step 510, the control system sets a rate of change based on the determined sleep stage for sleep portion X. Steps 508 and 510 are the same as, or similar to, steps 406 and 408 described above in connection with FIG. 4.

At step 512, the control system 390 causes the respiratory device 302 to change pressure of the pressurized air provided to the user 104 so that the pressure of the pressurized air changes at the set rate of change to a pressure value within the set pressure range.

At step 514, additional physiological data is received for a new sleep portion. That is, the sleep portion number is incremented → X=X+1. At step 516, the additional physiological data is analyzed to determine a sleep stage of the new sleep portion.

At step 518, the control system 390 determines whether the sleep stages for the new sleep portion and the previous sleep portion are the same. If the sleep stages for the sleep portions are the same, then the control system 390 increments the sleep portion number and receives more physiological data at step 514. If the sleep stages of the sleep portions differ, then the control system 390 sets a pressure range and a rate of change based on the determined sleep stage for the sleep portion at steps 508 and 510, respectively. The control system 390 can then cause the respiratory device 302 to change pressure of the pressurized air based on the set pressure range and the set rate of change for the determined sleep stage.

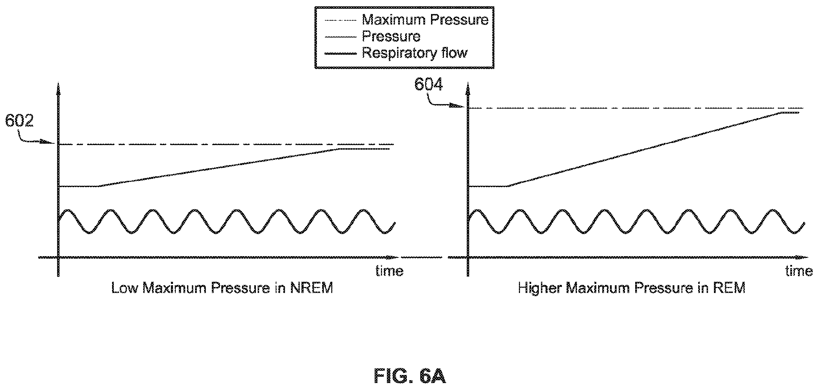

Having different pressure ranges and rates of change of pressure supplied by the respiratory device 302 provides a different type of therapy to the user 104. The respiratory device 302 behaves differently based on physiological data obtained on the user 104. FIGS. 6A-6D illustrate example responses of the respiratory device 302 under different sleep stages. Referring to FIG. 6A, an example where maximum pressure provided by a respiratory system differs between sleep stages is shown, according to some implementations of the present disclosure. In FIG. 6A, a maximum pressure 602 in an NREM sleep stage is lower than a maximum pressure 604 in a REM sleep stage. As such, the range of pressures for the NREM sleep stage is different than the range of pressures for the REM sleep stage.

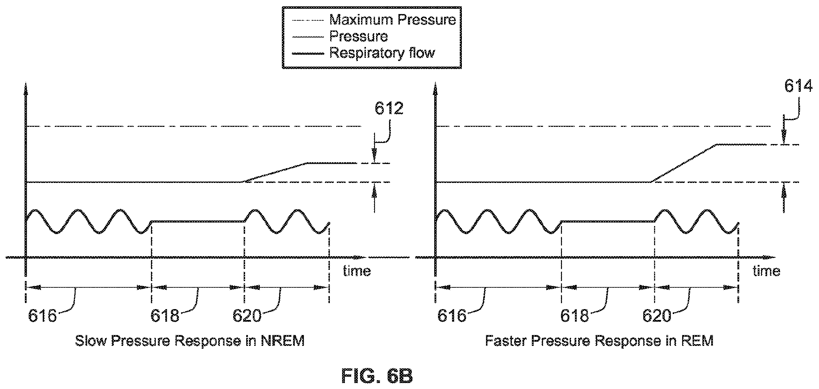

FIG. 6B illustrates an example where rate of increasing pressure in response to an apnea event differs between sleep stages, according to some implementations of the present disclosure. In FIG. 6B, a user (e.g., the user 104) has a regular respiratory flow during a period 616 then suffers an apnea during a period 618 and then resumes a regular respiratory flow during a period 620. After the apnea during the period 618, the pressure changes from a lower pressure to a higher pressure. But as indicated in FIG. 6B, the change 612 in pressure is smaller than the change 614 in pressure for an approximately equal time of change. Thus, in FIG. 6B, the rate of change is lower in an NREM sleep stage than the rate of change in a REM sleep stage.

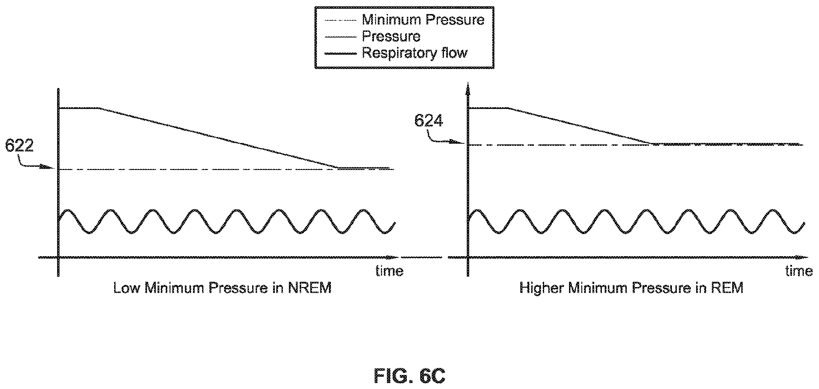

FIG. 6C illustrates an example where minimum pressure provided by a respiratory system differs between sleep stages, according to some implementations of the present disclosure. In FIG. 6A, a minimum pressure 622 in an NREM sleep stage is lower than a minimum pressure 624 in a REM sleep stage. As such, the range of pressures for the NREM sleep stage is different than the range of pressures for the REM sleep stage.

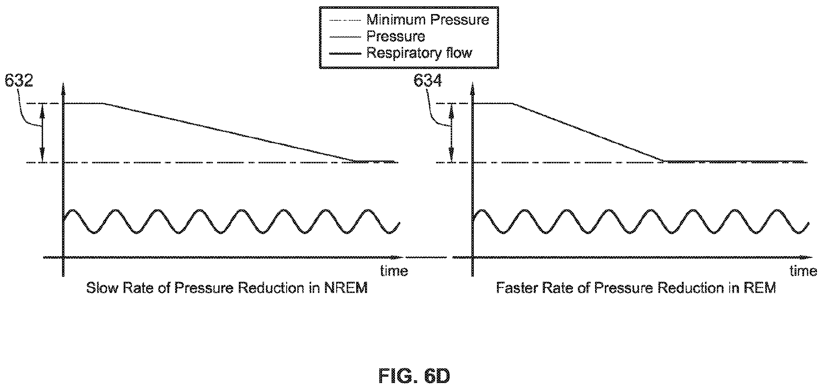

FIG. 6D illustrates an example where rate of decreasing pressure differs between sleep stages, according to some implementations of the present disclosure. Minimizing the pressure of the pressurized air provided to the user 104 can enhance comfort of the user 104 during a sleeping session. The control system 390 can reduce pressure in a sleep stage to its minimum pressure for a specific sleep stage, but the rate of change also differs within the sleep stage. As shown in FIG. 6D, the change 632 in pressure is comparable to the change 634 in pressure, but in an NREM sleep stage, the change 632 takes more time to complete than the change 634 in a REM sleep stage. As such, the NREM sleep stage is shown in this example to have a slower rate of pressure reduction than the REM sleep stage.

Although rate of change is used throughout the disclosure, rate of change can refer to a rate of increasing pressure and/or a rate of decreasing pressure. Within a sleep stage, the rate of increasing pressure can have a different magnitude than the rate of decreasing pressure. In some implementations, a rate of increasing pressure in an NREM sleep stage is not equal in magnitude to a rate of decreasing pressure in the NREM sleep stage. In some implementations, a rate of increasing pressure in a REM sleep stage is not equal in magnitude to a rate of decreasing pressure in the REM sleep stage.

Referring to FIG. 7, a flow diagram illustrating a method for monitoring and adjusting a configuration of a respiratory system (e.g., the respiratory device 302 and/or the system 300) based on a likelihood of its user (e.g., the user 104) having an apnea event, according to some implementations of the present disclosure. At step 702, the control system 390 receives physiological data associated with the user according to some implementations of the disclosure. Step 702 is the same as, or similar to, step 402 described above in connection with FIG. 4.

At step 704, the control system 390 analyzes the physiological data to determine a current sleep stage of the user 104 and/or a current breathing pattern of the user 104. The current sleep stage of the user can be determined as described elsewhere herein (e.g., as described in connection with step 404 of FIG. 4). The current breathing pattern of the user 104 can include a frequency of respiration flow, a shape of respiration flow, a volume of any snoring event, an amount of any breath flattening, a recent apnea event, or any combination thereof. In some implementations, the respiration flow can be determined by the respiratory device 302 as described elsewhere herein. When the respiratory flow is determined, its frequency and shape can be discerned from monitoring the respiratory flow for an amount of time, such as, for example, for five seconds, for ten seconds, etc. Breath flattening can also be determined from the shape of the respiratory flow where a departure of the shape from a sinusoid indicates a degree of flattening.

At step 706, the control system 390 determines a likelihood that the user 104 will experience an apnea event within a predetermined amount of time based at least in part on the determined current sleep stage and the determined current breathing pattern. The predetermined amount of time can be 20 seconds, 30 seconds, 1 minute, and so on. In some implementations, the control system 390 determines the likelihood via a machine learning algorithm that takes as inputs the received physiological data, a current blood alcohol level, a current position of the user, the determined current sleep stage of the user, the current breathing pattern of the user, the current pressure of the pressurized air, or any combination thereof.

In some implementations, the machine learning algorithm is trained with input data associated with a plurality of other users. The input data can include: (i) a body mass index (BMI) for each of the plurality of other users, (ii) sensor data associated with each of the plurality of other users, (iii) severity of apnea events for each of the plurality of other users, (iv) sex of each of the plurality of other users, (v) height of each of the plurality of other users, (vi) weights of each of the plurality of other users, or (vii) any combination of (i) through (vi).

At step 708, based at least in part on the determined likelihood, the control system 390 causes the respiratory device 302 to change, according to a rate of change based at least in part on the determined current sleep stage of the user, a current pressure of the supplied pressurized air to a second pressure. In some implementations, the respiratory device 302 changes the current pressure to the second pressure after waiting for a current apnea event to pass.

In some implementations, the respiratory device 302 incrementally adjusts the current pressure according to the rate of change. For each incremental adjustment, the control system 390 re-determines the likelihood that the user will experience another apnea event within the predetermined amount of time. The incremental adjustment can continue until the determined likelihood is below a predetermined threshold. For example, the control system 390 continually calculates the likelihood after each incremental adjustment until the likelihood is below 50%, below 40%, below 30%, below 20%, below 15%, below 10%, below 5%, etc. or below any other percentage.

In some implementations, machine learning can be used to learn a likelihood function that describes whether the user 104 will experience an apnea event. In a first step, the control system 390 can accumulate and store physiological data including historical physiological data and current physiological data. In a second step, the control system 390 can train a machine learning algorithm with the historical physiological data such that the machine learning algorithm is configured to receive as an input the current physiological data and determine as an output a likelihood that the user 104 will experience an apnea event within a predetermined amount of time.

In some implementations, the historical physiological data is divided by the control system 390 into different portions with each portion being associated with a sleep stage. For example, during a sleep session, the user 104 can cycle multiple times between relaxed wakefulness, light sleep, deep sleep and REM sleep stages. The historical physiological data can be divided into the number of different changes in sleep stage in some implementations such that there are multiple REM sleep stages or multiple deep sleep stages. The historical physiological data can also be divided into the specific groupings of sleep stages in some implementations such that for each sleep session, the multiple REM sleep stages are grouped as one REM sleep stage, the multiple deep sleep stages are grouped as one deep sleep stage, and so on. The sleep stage information can be used to train the machine learning algorithm.

In some implementations, the control system 390 trains the machine learning algorithm with one or more historical breathing patterns of the user, one or more historical pressures of the supplied pressurized air, one or more historical blood alcohol level of the user, one or more historical positions of the user, or any combination thereof. The historical breathing pattern of the user can include a frequency of respiration flow, a shape of respiration flow, a volume of any snoring event, an amount of any breath flattening, recent apnea events of the user, or any combination thereof.

In some implementations, the machine learning system is further trained by the control system 390 with data associated with a plurality of other users. The data can include (i) body mass index for each of the plurality of other users, (ii) sensor data associated with each of the plurality of other users, (iii) severity of apnea events for each of the plurality of other users, (iv) sex of each of the plurality of other users, (v) height of each of the plurality of other users, (vi) weights of each of the plurality of other users, or (vii) any combination of (i) through (vi).

In some implementations, the machine learning algorithm is further trained at a minimum pressure which is lower than the prescribed pressure. The minimum pressure is set at a value such that the minimum pressure is not therapeutic and the user 104 can experience apnea events without intervention from the respiratory device 302. The training of the machine learning algorithm at the minimum pressure allows apnea events to occur so that the control system 390 confirms certain features in the physiological data being present actually led to an apnea event occurring within a predetermined time. Training at the minimum pressure without intervention can avoid the case where a prescribed pressure prevents the machine learning algorithm from recording an apnea event. That is, the prescribed pressure may be enough to prevent an apnea so the control system 390 may not learn of certain features in the physiological data that can lead to apneas. Thus, training at the minimum pressure allows the control system 390 to identify more features in the physiological data that can indicate that an apnea will occur within a predetermined amount of time.

In some implementations, the control system 390 determines a current sleep stage of the user based on the current physiological data. The current sleep stage can then be used while determining the likelihood that the user 104 will experience an apnea event. For example, a pressure of supplied pressurized air may be adequate in a light sleep stage to prevent an apnea but based on the current sleep stage being REM sleep, the likelihood determined is higher than if the current sleep stage had been light sleep. Although REM sleep is used as an example, training with physiological data under different sleep stages allows the control system 390 to personalize and customize the likelihood function describing whether the user 104 will experience an apnea event within a predetermined amount of time. That way the control system 390 learns, for particular individuals, which sleep stages require higher pressures based on the determined likelihoods.

In some implementations, the control system 390 learns a range of pressures based on sleep stage by incrementally adjusting a current pressure of the supplied pressurized air according to a rate of change, and for each incremental adjustment, re-determining the likelihood that the user will experience another apnea event within the predetermined amount of time. The control system 390 can obtain lower bounds and upper bounds of the range of pressures for each sleep stage based on likelihoods calculated. For example, an acceptable likelihood can be set at a value below 20%, and the control system 390 can observe breathing pattern and current sleep stage of the user 104 and cause the respiratory device to adjust the pressure of the supplied pressurized air to keep the likelihood value below 20%. In some implementations, the range of pressures for keeping the likelihood below the 20% predetermined threshold is different from one sleep stage to another.

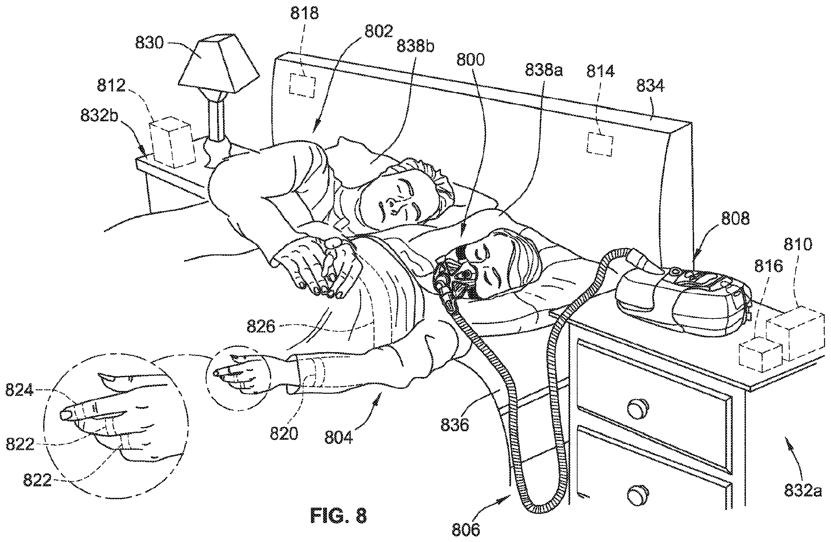

Referring to FIG. 8, an example placement of components for adjusting pressure of a respiratory device 808 in a bedroom setting, is shown according to some implementations of the present disclosure. The environment is a bedroom setting that includes a user 804, a bed partner 802, a bed with a mattress 836, pillows 838a and 838b, and a headboard 834. Next to the bed are two nightstands 832a and 832b, and a lamp 830 on the nightstand 832b. The user 804 is wearing a mask 800 connected via an air circuit or tubing 806 to the respiratory device 808. Non-contact sensing can be achieved with non-contact sensors like cameras, infrared cameras, motion sensors, radar sensors, sonar sensors, and/or microphones placed at positions 818, 814, 812, 816, and 810. Various other locations for the components are contemplated. For example, one or more cameras can be mounted in a ceiling of the room. One or more microphones, microphones and speakers for sonar, or transmitters and receivers for radar can be mounted to the headboard 834 and/or a wall of the room, etc. In some implementations, having multiple cameras or microphones at different locations in the room allows for multiple video angles and stereo sound which can allow for directly distinguishing and eliminating noise coming from the bed partner 802 relative to the user 804. In some implementations, contact sensors like PPG sensors, GSR sensors, ECG sensors, actigraphy sensors and so on can be placed on the user 804 at locations 820, 822, 824, and 826.

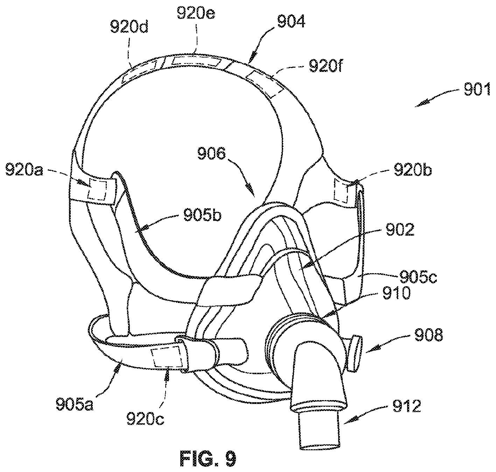

Referring to FIG. 9, a perspective view of a mask 901 that can be used with a respiratory device (e.g., the respiratory device 808) to is shown according to some implementations of the present disclosure. The mask 901 is the same as, or similar to, the mask 201 of FIG 2. The mask 901 includes a plenum chamber 902, a positioning or stabilizing structure 904 with strap segments 905a, 905b, and 905c, a cushion 906, an electronics interface 908, a connecting section 910, and a connection port 912. The mask 901 can include several locations for embedding sensors. For example, sensors or the like can be positioned at positions 920a, 920b, 920c, 920d, 920e, and/or 920f. Such sensors can include a microphone embedded in the mask 901 for use in determining a volume of snoring, EEG sensors for determining brainwaves, GSR sensors for determining skin resistance (perspiration), temperature sensors, and so on.

Exemplary embodiments
1. A method comprising:
   receiving, from a sensor, physiological data associated with a user;
   determining a first sleep stage of the user based on the received physiological data;
   based on the determined first sleep stage of the user, setting a range of pressures for a respiratory device to supply pressurized air, the respiratory device being coupled to a mask configured to engage the user during a sleep session to aid in directing the supplied pressurized air to an airway of the user; and
   based on the determined first sleep stage of the user, setting a rate of change of the pressurized air for the respiratory device to use when changing the supplied pressurized air from a first pressure within the range of pressures to a second pressure within the range of pressures.
2. The method of embodiment 1, further comprising:
   analyzing the physiological data to determine that the first sleep stage of the user changed from the first sleep stage to a second sleep stage that is different than the first sleep stage; and
   based at least in part on the determined second sleep stage of the user, setting a second range of pressures for the respiratory device to supply the pressurized air, the second range of pressures being different than the range of pressures.
3. The method of embodiment 2, further comprising:
   based at least in part on the determined second sleep stage of the user, setting a second rate of change of the pressurized air for the respiratory device to use when changing the supplied pressurized air from the second pressure to a third pressure within the second range of pressures.
4. The method of any one of embodiments 1 to 3, wherein the determined first sleep stage is wakefulness, relaxed wakefulness, light sleep, deep sleep, or REM sleep.
5. The method of embodiment 4, wherein a magnitude of the set rate of change of the pressurized air is higher in REM sleep than in any of relaxed wakefulness, light sleep, or deep sleep.
6. The method of embodiment 4 or embodiment 5, wherein: (i) a maximum pressure of the range of pressures is higher in REM sleep than in any of relaxed wakefulness, light sleep, or deep sleep, and (ii) a minimum pressure of the range of pressures is higher in REM sleep than in any of relaxed wakefulness, light sleep, or deep sleep.
7. The method of embodiment 4 or embodiment 5, wherein a maximum pressure of the range of pressures is higher in deep sleep than in REM sleep.
8. The method of any one of claims 1 to 7, wherein the physiological data includes breath analysis, airway resistance, blood flow to brain, blood pressure, skin temperature, core temperature, brain activity, heart rate, muscle tone, respiration rate, sexual arousal, sympathetic nerve activity, blood alcohol level, blood oxygenation, expired carbon dioxide, body movement, chest movement, limb movement, body position, or any combination thereof.
9. The method of any one of embodiments 1 to 8, wherein the sensor is coupled to the mask and is configured to measure brain activity.
10. The method of any one of embodiments 1 to 9, wherein the sensor includes one or more microphones, one or more speakers, one or more radio frequency (RF) transmitters, one or more RF receivers, one or more ultra wide band (UWB) transmitters, one or more UWB receivers, one or more flow sensors, one or more pressure sensors, one or more actigraphy sensors, one or more motion sensors, one or more galvanic sensors, one or more temperature sensors, one or more PPG sensors, one or more sphygmomanometer sensors, one or more pulse sensors, one or more oximetry sensors, or any combination thereof.
11. The method of any one of embodiments 1 to 10, further comprising:
   measuring an amount of breath flattening of the user occurring in a first breathing cycle; and
   based at least in part on the amount of breath flattening exceeding a predetermined threshold, causing the respiratory device to change the supplied pressurized air from the first pressure to the second pressure according to the set rate of change of the pressurized air.
12. The method of any one of embodiments 1 to 10, further comprising:
   measuring a magnitude of snoring of the user occurring in a first breathing cycle; and based at least in part on the magnitude of snoring exceeding a predetermined threshold,
   causing the respiratory device to change the supplied pressurized air from the first pressure to the second pressure according to the set rate of change of the pressurized air.
13. The method of any one of embodiments 1 to 10, further comprising:
   detecting occurrence of an apnea of the user occurring in a first breathing cycle; and
   after the apnea has occurred, causing the respiratory device to change the supplied pressurized air from the first pressure to the second pressure according to the set rate of change of the pressurized air.
14. A method comprising:
   receiving, from a sensor, physiological data associated with a user;
   analyzing the physiological data to determine (i) a current sleep stage of the user and (ii) a current breathing pattern of the user;
   determining a likelihood that the user will experience an apnea event within a predetermined amount of time based at least in part on the determined current sleep stage and the determined current breathing pattern; and
   based at least in part on the determined likelihood, causing a respiratory device supplying pressurized air to change a current pressure of the supplied pressurized air to a second pressure, the respiratory device changing the current pressure according to a rate of change based at least in part on the determined current sleep stage of the user, and the respiratory device being coupled to a mask configured to engage the user to aid in directing the supplied pressurized air to an airway of the user.
15. The method of embodiment 14, wherein the causing the respiratory device to change the current pressure of the supplied pressurized air includes incrementally adjusting the current pressure according to the rate of change and, for each incremental adjustment, re-determining the likelihood that the user will experience another apnea event within the predetermined amount of time.
16. The method of embodiment 15, wherein the incrementally adjusting occurs until the redetermined likelihood is below a predetermined threshold.
17. The method of any one of embodiments 14 to 16, wherein the likelihood that the user will experience an apnea event is determined via a machine learning algorithm taking as inputs the received physiological data, the determined current sleep stage of the user, the determined current breathing pattern of the user, the current pressure of the pressurized air, a current blood alcohol level of the user, a current position of the user, or any combination thereof.
18. The method of embodiment 17, wherein the machine learning algorithm further takes as inputs data associated with a plurality of other users, the data including (i) a body mass index (BMI) for each of the plurality of other users, (ii) sensor data associated with each of the plurality of other users, (iii) severity of apnea events for each of the plurality of other users, (iv) sex of each of the plurality of other users, (v) height of each of the plurality of other users, (vi) weights of each of the plurality of other users, or (vii) any combination of (i) through (vi).
19. The method of any one of claims 14 to 18, wherein the determined current breathing pattern of the user includes a frequency of respiration flow, a shape of respiration flow, a volume of any snoring event, an amount of any breath flattening, recent apnea events of the user, or any combination thereof.
20. The method of any one of embodiments 14 to 19, wherein the determined sleep stage is a first sleep stage that is different from a second sleep stage, the second pressure being within a first range of pressures corresponding to the first sleep stage, and the first range of pressures being different from a second range of pressures corresponding to the second sleep stage.
21. The method of embodiment 20, wherein in the first sleep stage, the rate of change includes a first rate of change for increasing pressure of the supplied pressurized air and a second rate of change for decreasing pressure the supplied pressurized air, the first rate of change and the second rate of change being the same or different.
22. The method of embodiment 20 or embodiment 21, wherein in the second sleep stage, the rate of change includes a third rate of change for increasing pressure of the supplied pressurized air and a fourth rate of change for decreasing pressure of the supplied pressurized air, the third rate of change and the fourth rate of change being the same or different.
23. The method of embodiment 22, wherein the each of the first rate of change, the second rate of change, the third rate of change, and the fourth rate of change has a different magnitude.
24. The method of embodiment 21 or embodiment 23, further comprising:
   causing the respiratory device to reduce the current pressure of the supplied pressurized air from the second pressure to a third pressure according to the second rate of change.
25. The method of any one of embodiments 21 to 24, wherein the first rate of change and the second rate of change have different magnitudes.
26. The method of any one of embodiments 20 to 25, wherein a maximum pressure of the first range of pressures is higher than a maximum pressure of the second range of pressures.
27. The method of any one of embodiments 14 to 26, wherein the causing the respiratory device to change the current pressure includes waiting for a current apnea event to pass and then causing the respiratory device to change the current pressure of the supplied pressurized air to the second pressure.
28. The method of embodiment 14, wherein the physiological data includes historical physiological data and current physiological data, the method further comprising:
   training a machine learning algorithm with the historical physiological data such that the machine learning algorithm is configured to receive as an input the current physiological data and determine as an output the likelihood that the user will experience the apnea event within the predetermined amount of time.
29. The method of embodiment 28, wherein the control system is further configured to determine, for each of a plurality of portions of the historical physiological data, a corresponding sleep stage of the user, wherein the current sleep stage of the user is determined based at least in part on the current physiological data.
30. The method of embodiment 29, wherein the machine learning algorithm is further trained with the determined sleep stage of the user for each of the plurality of portions of the historical physiological data.
31. The method of embodiment 29 or embodiment 30, wherein each of the plurality of portions of the historical physiological data is associated with the user being in a single sleep stage.
32. The method of embodiment 28, further comprising:
   learning a customized range of pressures for the user for each of a plurality of sleep stages by incrementally adjusting the current pressure of the supplied pressurized air according to the rate of change, and for each incremental adjustment, re-determining the likelihood that the user will experience another apnea event within the predetermined amount of time.
33. The method of embodiment 32, wherein each incremental adjustment increases the current pressure of the supplied pressurized air to determine an upper bound of the customized range of pressures for the current sleep stage.
34. The method of embodiment 32, wherein each incremental adjustment decreases the current pressure of the supplied pressurized air to determine a lower bound of the customized range of pressures for the current sleep stage.
35. The method of any one of embodiments 28 to 34, wherein the machine learning algorithm is further trained with one or more historical breathing patterns of the user, one or more historical pressures of the supplied pressurized air, one or more historical blood alcohol level of the user, one or more historical positions of the user, or any combination thereof.
36. The method of embodiment 35, wherein the historical breathing pattern of the user includes a frequency of respiration flow, a shape of respiration flow, a volume of any snoring event, an amount of any breath flattening, recent apnea events of the user, or any combination thereof.
37. The method of any one of embodiments 28 to 36, wherein the machine learning algorithm is further trained with data associated with a plurality of other users, the data including (i) body mass index for each of the plurality of other users, (ii) sensor data associated with each of the plurality of other users, (iii) severity of apnea events for each of the plurality of other users, (iv) sex of each of the plurality of other users, (v) height of each of the plurality of other users, (vi) weights of each of the plurality of other users, or (vii) any combination of (i) through (vi).
38. The method of any one of embodiments 28 to 37, wherein the machine learning algorithm is further trained at a minimum pressure, the minimum pressure being lower than a prescribed pressure.
39. A system comprising:
   a control system including one or more processors; and
   a memory having stored thereon machine readable instructions;
   wherein the control system is coupled to the memory, and the method of any one of embodiments 1 to 38 is implemented when the machine executable instructions in the memory are executed by at least one of the one or more processors of the control system.
40. A system for identifying a potential candidate, the system including a control system configured to implement the method of any one of embodiments 1 to 38.
41. A computer program product comprising instructions which, when executed by a computer, cause the computer to carry out the method of any one of embodiments 1 to 38.
42. The computer program product of embodiment 41, wherein the computer program product is a non-transitory computer readable medium.
43. A system comprising:
   a respiratory device configured to supply pressurized air;
   a mask coupled to the respiratory device via a tube, the mask being configured to engage a user during a sleep session to aid in directing the supplied pressurized air to an airway of the user;
   a sensor configured to generate physiological data associated with the user;
   a memory storing machine-readable instructions; and
   a control system including one or more processors configured to execute the machine-readable instructions to:
      analyze the physiological data to determine a first sleep stage of the user;
      based on the determined first sleep stage of the user, set a range of pressures for the respiratory device to supply the pressurized air; and
      based on the determined first sleep stage of the user, set a rate of change of the pressurized air for the respiratory device to use when changing the supplied pressurized air from a first pressure within the range of pressures to a second pressure within the range of pressures.
44. The system of embodiment 43, wherein the control system is further configured to:
   analyze the physiological data to determine that the first sleep stage of the user changed from the first sleep stage to a second sleep stage that is different than the first sleep stage;
   based at least in part on the determined second sleep stage of the user, set a second range of pressures for the respiratory device to supply the pressurized air, the second range of pressures being different than the range of pressures.
45. The system of embodiment 44, wherein the control system is further configured to:
   based at least in part on the determined second sleep stage of the user, set a second rate of change of the pressurized air for the respiratory device to use when changing the supplied pressurized air from the second pressure to a third pressure within the second range of pressures.
46. The system of any one of embodiments 43 to 45, wherein the determined first sleep stage is wakefulness, relaxed wakefulness, light sleep, deep sleep, or REM sleep.
47. The system of embodiment 46, wherein a magnitude of the set rate of change of the pressurized air is higher in REM sleep than in any of relaxed wakefulness, light sleep, or deep sleep.
48. The system of embodiment 46 or embodiment 47, wherein: (i) a maximum pressure of the range of pressures is higher in REM sleep than in any of relaxed wakefulness, light sleep, or deep sleep, and (ii) a minimum pressure of the range of pressures is higher in REM sleep than in any of relaxed wakefulness, light sleep, or deep sleep.
49. The system of embodiment 46 or embodiment 47, wherein a maximum pressure of the range of pressures is higher in deep sleep than in REM sleep.
50. The system of any one of embodiments 43 to 49, wherein the physiological data generated by the sensor includes breath analysis, airway resistance, blood flow to brain, blood pressure, skin temperature, core temperature, brain activity, heart rate, muscle tone, respiration rate, sexual arousal, sympathetic nerve activity, blood alcohol level, blood oxygenation, expired carbon dioxide, body movement, chest movement, limb movement, body position, or any combination thereof.
51. The system of any one of embodiments 43 to 50, wherein the sensor is coupled to the mask and is configured to measure brain activity.
52. The system of any one of embodiments 43 to 51, wherein the sensor includes one or more microphones, one or more speakers, one or more radio frequency (RF) transmitters, one or more RF receivers, one or more ultra wide band (UWB) transmitters, one or more UWB receivers, one or more flow sensors, one or more pressure sensors, one or more actigraphy sensors, one or more motion sensors, one or more galvanic sensors, one or more temperature sensors, one or more PPG sensors, one or more sphygmomanometer sensors, one or more pulse sensors, one or more oximetry sensors, or any combination thereof.
53. The system of any one of embodiments 43 to 52, wherein the control system is further configured to:
   measure an amount of breath flattening of the user occurring in a first breathing cycle, and
   based at least in part on the amount of breath flattening exceeding a predetermined threshold, cause the respiratory device to change the supplied pressurized air from the first pressure to the second pressure according to the set rate of change of the pressurized air.
54. The system of any one of embodiments 43 to 52, wherein the control system is further configured to:
   measure a magnitude of snoring of the user occurring in a first breathing cycle, and
   based at least in part on the magnitude of snoring exceeding a predetermined threshold, cause the respiratory device to change the supplied pressurized air from the first pressure to the second pressure according to the set rate of change of the pressurized air.
55. The system of any one of embodiments 43 to 52, wherein the control system is further configured to:
   detect the occurrence of an apnea of the user occurring in a first breathing cycle, and
   after the apnea has occurred, cause the respiratory device to change the supplied pressurized air from the first pressure to the second pressure according to the set rate of change of the pressurized air.
56. A system comprising:
   a respiratory device configured to supply pressurized air;
   a mask coupled to the respiratory device via a tube, the mask being configured to engage a user during a sleep session to aid in directing the supplied pressurized air to an airway of the user;
   a sensor configured to generate physiological data associated with the user;
   a memory storing machine-readable instructions; and
   a control system including one or more processors configured to execute the machine-readable instructions to:
      analyze the physiological data to determine (i) a current sleep stage of the user and (ii) a current breathing pattern of the user;
      determine a likelihood that the user will experience an apnea event within a predetermined amount of time based at least in part on the determined current sleep stage and the determined current breathing pattern; and
      based at least in part on the determined likelihood, cause the respiratory device to change, according to a rate of change based at least in part on the determined current sleep stage of the user, a current pressure of the supplied pressurized air to a second pressure.
57. The system of embodiment 56, wherein the causing the respiratory device to change the current pressure of the supplied pressurized air includes incrementally adjusting the current pressure according to the rate of change and, for each incremental adjustment, re-determining the likelihood that the user will experience another apnea event within the predetermined amount of time.
58. The system of embodiment 57, wherein the incrementally adjusting occurs until the redetermined likelihood is below a predetermined threshold.
59. The system of any one of embodiments 55 to 57, wherein the likelihood that the user will experience an apnea event is determined via a machine learning algorithm taking as inputs the generated physiological data, the determined current sleep stage of the user, the current breathing pattern of the user, the current pressure of the pressurized air, a current blood alcohol level of the user, a current position of the user, or any combination thereof.
60. The system of embodiment 59, wherein the machine learning algorithm further takes as inputs data associated with a plurality of other users, the data including (i) a body mass index (BMI) for each of the plurality of other users, (ii) sensor data associated with each of the plurality of other users, (iii) severity of apnea events for each of the plurality of other users, (iv) sex of each of the plurality of other users, (v) height of each of the plurality of other users, (vi) weights of each of the plurality of other users, or (vii) any combination of (i) through (vi).
61. The system of any one of claims 56 to 60, wherein the determined current breathing pattern of the user includes a frequency of respiration flow, a shape of respiration flow, a volume of any snoring event, an amount of any breath flattening, recent apnea events of the user, or any combination thereof.
62. The system of any one of embodiments 56 to 61, wherein the determined sleep stage is a first sleep stage that is different from a second sleep stage, the second pressure being within a first range of pressures corresponding to the first sleep stage, and the first range of pressures being different from a second range of pressures corresponding to the second sleep stage.
63. The system of embodiment 62, wherein in the first sleep stage, the rate of change includes a first rate of change for increasing pressure of the supplied pressurized air and a second rate of change for decreasing pressure the supplied pressurized air, the first rate of change and the second rate of change being the same or different.
64. The system of embodiment 62 or embodiment 63, wherein in the second sleep stage, the rate of change includes a third rate of change for increasing pressure of the supplied pressurized air and a fourth rate of change for decreasing pressure of the supplied pressurized air, the third rate of change and the fourth rate of change being the same or different.
65. The system of embodiment 64, wherein the each of the first rate of change, the second rate of change, the third rate of change, and the fourth rate of change has a different magnitude.
66. The system of embodiment 63 or embodiment 65, wherein the control system is further configured to cause the respiratory device to reduce the current pressure of the supplied pressurized air from the second pressure to a third pressure according to the second rate of change.
67. The system of any one of embodiments 63 to 66, wherein the first rate of change and the second rate of change have different magnitudes.
68. The system of any one of embodiments 63 to 66, wherein a magnitude of the first rate of change is higher than a magnitude of the second rate of change.
69. The system of any one of embodiments 63 to 66, wherein a magnitude of the first rate of change is less than a magnitude of the second rate of change.
70. The system of any one of embodiments 63 to 66, wherein a magnitude of the second rate of change is at least two times a magnitude of the first rate of change.
71. The system of any one of embodiments 62 to 70, wherein a maximum pressure of the first range of pressures is higher than a maximum pressure of the second range of pressures.
72. The system of any one of embodiments 56 to 71, wherein the causing the respiratory device to change the current pressure includes waiting for a current apnea event to pass and then causing the respiratory device to change the current pressure of the supplied pressurized air to the second pressure.
73. A system comprising:
   a respiratory device configured to supply pressurized air;
   a mask coupled to the respiratory device via a tube, the mask being configured to engage a user during a sleep session to aid in directing the supplied pressurized air to an airway of the user;
   a sensor configured to generate physiological data associated with the user;
   a memory storing machine-readable instructions; and
   a control system including one or more processors configured to execute the machine-readable instructions to:
      accumulate the physiological data, the physiological data including historical physiological data and current physiological data; and
      train a machine learning algorithm with the historical physiological data such that the machine learning algorithm is configured to receive as an input the current physiological data and determine as an output a likelihood that the user will experience an apnea event within a predetermined amount of time.
74. The system of embodiment 73, wherein the control system is further configured to determine, for each of a plurality of portions of the historical physiological data, a corresponding sleep stage of the user.
75. The system of embodiment 74, wherein the machine learning algorithm is further trained with the determined sleep stage of the user for each of the plurality of portions of the historical physiological data.
76. The system of embodiment 74 or embodiment 75, wherein each of the plurality of portions of the historical physiological data is associated with the user being in a single sleep stage.
77. The system of any one of embodiments 73 to 76, wherein the control system is further configured to determine, for the current physiological data, a current sleep stage of the user.
78. The system of embodiment 77, wherein the determined likelihood is based at least in part on the current sleep stage of the user.
79. The system of embodiment 73, wherein the control system is further configured to learn a customized range of pressures for the user for each of a plurality of sleep stages by incrementally adjusting a current pressure of the supplied pressurized air according to a rate of change, and for each incremental adjustment, re-determining the likelihood that the user will experience another apnea event within the predetermined amount of time.
80. The system of embodiment 79, wherein each incremental adjustment increases the current pressure of the supplied pressurized air to determine an upper bound of the customized range of pressures for the current sleep stage.
81. The system of embodiment 79, wherein each incremental adjustment decreases the current pressure of the supplied pressurized air to determine a lower bound of the customized range of pressures for the current sleep stage.
82. The system of any one of embodiments 73 to 81, wherein the machine learning algorithm is further trained with one or more historical breathing patterns of the user, one or more historical pressures of the supplied pressurized air, one or more historical blood alcohol level of the user, one or more historical positions of the user, or any combination thereof.
83. The system of embodiment 82, wherein the historical breathing pattern of the user includes a frequency of respiration flow, a shape of respiration flow, a volume of any snoring event, an amount of any breath flattening, recent apnea events of the user, or any combination thereof.
84. The system of any one of embodiments 73 to 83, wherein the machine learning algorithm is further trained with data associated with a plurality of other users, the data including (i) body mass index for each of the plurality of other users, (ii) sensor data associated with each of the plurality of other users, (iii) severity of apnea events for each of the plurality of other users, (iv) sex of each of the plurality of other users, (v) height of each of the plurality of other users, (vi) weights of each of the plurality of other users, or (vii) any combination of (i) through (vi).
85. The system of any one of embodiments 73 to 84, wherein the machine learning algorithm is further trained at a minimum pressure, the minimum pressure being lower than a prescribed pressure.
86. A method of supplying pressurized air to a user of a respiratory device, the method comprising:
   supplying, via the respiratory device, pressurized air at a first pressure to the user via a mask coupled to the respiratory device, the first pressure being within a first range of pressures;
   generating, via a sensor, first physiological data associated with a first portion of a sleep session of the user;
   determining a first sleep stage of the user based at least in part on the first physiological data;
   based on the determined first sleep stage of the user, changing the first pressure according to a first rate of change to a second pressure such that the second pressure of the supplied pressurized air is within the first range of pressures;
   generating, via the sensor, second physiological data associated with a second portion of the sleep session of the user;
   determining a second sleep stage of the user based at least in part on the second physiological data, the second sleep stage being different than the first sleep stage; and
   based on the determined second sleep stage of the user, changing the second pressure according to a second rate of change to a third pressure such that the third pressure of the supplied pressurized air is within a second range of pressures that is different than the first range of pressures, the first rate of change and the second rate of change having different magnitudes.
87. The method of embodiment 86, wherein a maximum pressure of the first range of pressures is higher than a maximum pressure of the second range of pressures.
88. The method of embodiment 86 or embodiment 87, wherein the first range of pressures partially overlaps with the second range of pressures.
89. The method of any one of embodiments 86 to 88, further comprising:
   generating, via the sensor, third physiological data associated with a third portion of the sleep session of the user;
   determining a third sleep stage of the user based at least in part on the third physiological data;
   determining a likelihood that the user will experience an apnea event during the third sleep stage and within a predetermined amount of time.
90. The method of embodiment 89, further comprising, based at least in part on the determined likelihood, changing, according to a third rate of change, the third pressure of the supplied pressurized air to a fourth pressure.
91. The method of embodiment 90, wherein the second rate of change and the third rate of change have different magnitudes.

While the present disclosure has been described with reference to one or more particular implementations, those skilled in the art will recognize that many changes may be made thereto without departing from the spirit and scope of the present disclosure. Each of these embodiments and implementations and obvious variations thereof is contemplated as falling within the spirit and scope of the present disclosure, which is set forth in the claims that follow.

## Claims

1. A system (300) comprising:
a control system (390) including one or more processors;
a physiological sensor;
a respiratory device (108, 302); and
a memory (380) having stored thereon machine-readable instructions;
wherein the control system (390) is coupled to the memory (380), the physiological sensor, and the respiratory device (108, 302), and wherein the one or more processors of the control system (390) are configured to execute the machine-readable instructions to:
receive (402), from the physiological sensor, physiological data associated with a user;
determine (404) a first sleep stage of the user based on the received physiological data;
based at least in part on the determined first sleep stage of the user, set (406) a range of pressures for the respiratory device (108, 302) to supply pressurized air, the respiratory device (108, 302) being coupled to a mask (100, 201, 310) configured to engage the user during a sleep session to aid in directing the supplied pressurized air to an airway of the user; and
based at least in part on the determined first sleep stage of the user, set (406) a rate of change of the pressurized air for the respiratory device (108, 302) to use when changing the supplied pressurized air between a first pressure within the range of pressures and a second pressure within the range of pressures.

2. The system of claim 1, wherein the one or more processors of the control system (390) are further configured to execute the machine-readable instructions to:
analyze the physiological data to determine that the first sleep stage of the user changed from the first sleep stage to a second sleep stage that is different than the first sleep stage;
based at least in part on the determined second sleep stage of the user, set a second range of pressures for the respiratory device (108, 302) to supply the pressurized air, the second range of pressures being different than the range of pressures; and
based at least in part on the determined second sleep stage of the user, set a second rate of change of the pressurized air for the respiratory device (108, 302) to use when changing the supplied pressurized air from the second pressure to a third pressure within the second range of pressures.

3. The system of claim 1 or claim 2, wherein the determined first sleep stage is wakefulness, relaxed wakefulness, light sleep, deep sleep, or REM sleep, and wherein:
(i) a magnitude of the set rate of change of the pressurized air is higher in REM sleep than in any of relaxed wakefulness, light sleep, or deep sleep,
(ii) a maximum pressure of the range of pressures is higher in REM sleep than in any of relaxed wakefulness, light sleep, or deep sleep,
(iii) a minimum pressure of the range of pressures is higher in REM sleep than in any of relaxed wakefulness, light sleep, or deep sleep, or
(iv) any combination of (i)-(iii).

4. The system of any one of claims 1 to 3, wherein:
the physiological data includes breath analysis, airway resistance, blood flow to brain, blood pressure, skin temperature, core temperature, brain activity, heart rate, muscle tone, respiration rate, sexual arousal, sympathetic nerve activity, blood alcohol level, blood oxygenation, expired carbon dioxide, body movement, chest movement, limb movement, body position, or any combination thereof; and/or
the physiological sensor is coupled to the mask (100, 201, 310) and is configured to measure brain activity, optionally wherein the physiological sensor includes one or more microphones, one or more speakers, one or more radio frequency (RF) transmitters, one or more RF receivers, one or more ultra wide band (UWB) transmitters, one or more UWB receivers, one or more flow sensors, one or more pressure sensors, one or more actigraphy sensors, one or more motion sensors, one or more galvanic sensors, one or more temperature sensors, one or more PPG sensors, one or more sphygmomanometer sensors, one or more pulse sensors, one or more oximetry sensors, or any combination thereof.

5. The system of any one of claims 1 to 4, wherein the one or more processors of the control system (390) are further configured to execute the machine-readable instructions to:
learn which sleep stages of the user in which the user is more likely to experience apneas; and
determine a range of pressures, a rate of change of the pressurized air, or both, for each sleep stage based on the learning.

6. The system of any one of claims 1 to 5, wherein setting the range of pressures includes determining a maximum pressure, a minimum pressure, or both, based at least in part on the determined sleep stage of the user.

7. The system of any one of claims 1 to 6, wherein the pressure range for the first sleep stage is specific to the user.

8. The system of any one of claims 1 to 7, wherein the rate of change of the pressurized air is for the respiratory device (108, 302) to use when increasing a pressure of the pressurized air in response to an apnea event, when decreasing the pressure of the pressurized air, or both.

9. The system of any one of claims 1 to 8, wherein setting the rate of change of the pressurized air includes setting a first rate of change for increasing the pressure of the pressurized air and a second rate of change for decreasing the pressure of the pressurized air, and wherein for the determined first sleep stage of the user, the first rate of change is not equal in magnitude to the second rate of change.

10. The system of any one of claims 1 to 9, wherein determining (404) a first sleep stage of the user based on the received physiological data includes determining whether the first sleep stage of the user is a wakefulness stage, a rapid eye movement (REM) sleep stage, or a non-REM sleep stage, and wherein setting the range of pressures of the pressurized air includes setting a first range of pressures in a response to the first sleep stage being a REM sleep stage and a second range of pressures in response to the first sleep stage being a non-REM sleep stage, the first range of pressures being different than the second range of pressures.

11. The system of claim 10, wherein the first range of pressures includes a higher maximum pressure than the second range of pressures such that in response to an apnea event, the respiratory device can increase the pressure of the pressurized air higher when the user is in REM sleep than when the user is in non-REM sleep.

12. The system of any one of claims 1 to 11, wherein determining (404) a first sleep stage of the user based on the received physiological data includes determining whether the first sleep stage of the user is a wakefulness stage, a rapid eye movement (REM) sleep stage, or a non-REM sleep stage, and wherein setting the rate of change of the pressurized air includes setting a first rate of change in a response to the first sleep stage being a REM sleep stage and a second rate of change in response to the first sleep stage being a non-REM sleep stage, the first rate of change being different than the second rate of change.

13. The system of claim 12, wherein the first rate of change is larger than the second rate of change such that in response to an identical apnea event in REM sleep and non-REM sleep, the respiratory device increases the pressure of the pressurized air more quickly in REM sleep than in non-REM sleep.

14. A method comprising:
receiving (402), from a sensor, physiological data associated with a user;
determining (404) a first sleep stage of the user based on the received physiological data;
based at least in part on the determined first sleep stage of the user, setting (406) a range of pressures for a respiratory device (108, 302) to supply pressurized air, the respiratory device (108, 302) being coupled to a mask (100, 201, 310) configured to engage the user during a sleep session to aid in directing the supplied pressurized air to an airway of the user; and
based at least in part on the determined first sleep stage of the user, setting (406) a rate of change of the pressurized air for the respiratory device (108, 302) to use when changing the supplied pressurized air between a first pressure within the range of pressures and a second pressure within the range of pressures.

15. A computer program product comprising instructions which, when executed by a computer, cause the computer to carry out the method of claim 14, optionally wherein the computer program product is a non-transitory computer readable medium.
